# EUROPEAN PATENT APPLICATION

(11) **EP 4 516 767 A1**
(43) Date of publication of application: **05.03.2025**
(21) Application number: 23796295.6
(22) Date of filing: 24.04.2023
(51) Int. Cl.: C07B 61/00, C07D 209/44, C07D 209/50, C09B 57/04, C09B 67/20

(54) **ISOINDOLINE COMPOUND**

(30) Priority: 27.04.2022 JP 2022073265
(71) Applicant: DIC Corporation, Tokyo 174-8520 (JP)
(72) Inventor: FOO Siongwan, Tokyo 174-8520 (JP); EBATO Hiroshi, Tokyo 174-8520 (JP)
(74) Representative: TBK
(86) International application number: PCT/JP2023/016032
(87) International publication number: WO 2023/210555

(57) **Abstract**

Provided is a biomass-derived isoindoline compound (such as KII or DII) which is safe and can be obtained in a clean and carbon neutral approach. An isoindoline compound containing a radioactive carbon atom ¹⁴C and being represented by the following formula (I): 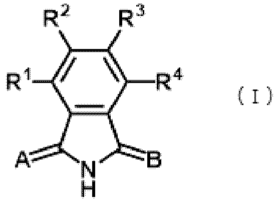 in which, in the formula (I), A and B each independently represent an oxygen atom or NH, and R¹ to R⁴ each independently represent a hydrogen atom, a linear or branched alkyl group having 1 to 12 carbon atoms, a fluorine atom, a chlorine atom, a bromine atom, an iodine atom, or a phenyl group which is optionally substituted with an alkyl group, wherein R² and R³ optionally undergo ring closure to form a 5- to 8-membered ring.

## Description

### TECHNICAL FIELD

The present invention relates to an isoindoline compound, a composition including the isoindoline compound, and a method for producing the isoindoline compound.

### BACKGROUND ART

KII (3-iminoisoindolin-1-one) is an important intermediate that can be used in synthesis of Y173 (isoindoline yellow pigment), and, meanwhile, DII (1,3-diiminoisoindoline) is an important intermediate for producing existing pigments, such as Y139 and Y185 (isoindoline yellow pigment), nonmetal phthalocyanine, and metal phthalocyanine.

KII and DII are an intermediate that is important not only for producing existing pigments but also for synthesizing a novel isoindoline pigment and dye and further bioactive isoindolines having properties of an anti-tumor agent, an antioxidant, an anti-fungus agent, or the like.

In a conventional synthesis method for KII or DII, mainly, phthalonitrile is used as a starting raw material (see, for example, NPLs 1 and 2), and further, with respect to the starting raw material, phthalic anhydride (see, for example, PTLs 1 and 2), methyl 2-cyanobenzoate, phthalimidate dimethyl hydrochloride, and the like have been reported.

However, phthalonitrile is harmful to human body and adversely affects the environment. Further, generally, the source of supply of phthalonitrile is only a petroleum resource.

In recent years, from the viewpoint of sustainable development, reduction of carbon dioxide emissions, and safety, it is more likely that the petroleum-derived raw materials are switched to a biomass-derived raw material, and that harmful chemical substances are replaced by those having lower toxicity.

With respect to the KII and DII, it is desired to provide biomass-derived KII and DII.

### CITATION LIST

### PATENT LITERATURE

PTL 1: JPH07-97366A
PTL 2: JPH07-330729A

### NON PATENT LITERATURE

NPL 1: Adv. Synth. Catal. 2008 350 1 135-142
NPL 2: Chem. Lett. 1984 8 1423-1426

### SUMMARY OF INVENTION

### TECHNICAL PROBLEM

An object of the present invention is to provide a biomass-derived isoindoline compound (such as KII or DII) which is safe and can be obtained in a clean and carbon neutral approach.

### SOLUTION TO PROBLEM

The present inventor has made studies with a view toward solving the above-mentioned problems. As a result, it has been found that biomass-derived furan and biomass-derived maleic anhydride are used as raw materials and subjected to the Diels-Alder (DA) reaction to obtain a DA intermediate, and the DA intermediate is subjected to dehydration and a ring opening reaction, and, on the final stage, part of or all of the oxygen atoms of the resultant intermediate are exchanged by nitrogen, so that bio KII or bio DII containing a radioactive carbon atom ¹⁴C can be obtained, and the present invention has been completed.

Specifically, the present invention includes the following embodiments.
[1] An isoindoline compound containing a radioactive carbon atom ¹⁴C and being represented by the following formula (I): 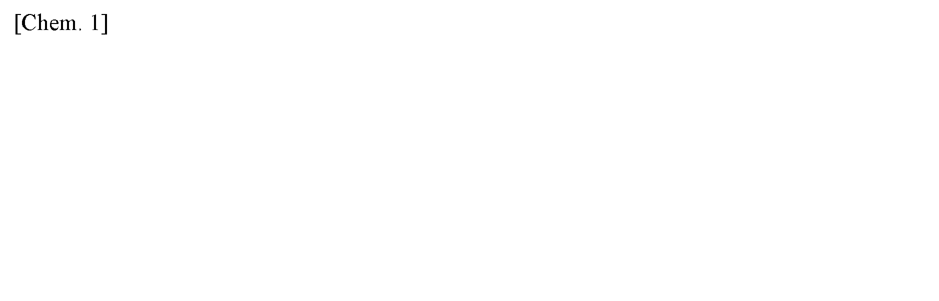 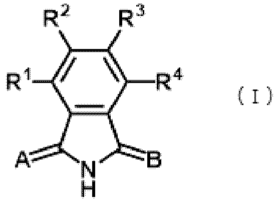 in which, in the formula (I), A and B each independently represent an oxygen atom or NH, and R¹ to R⁴ each independently represent a hydrogen atom, a linear or branched alkyl group having 1 to 12 carbon atoms, a fluorine atom, a chlorine atom, a bromine atom, an iodine atom, or a phenyl group which is optionally substituted with an alkyl group, in which R² and R³ optionally undergo ring closure to form a 5- to 8-membered ring.
[2] The compound according to item [1] above, in which the pMC (percent modern carbon) of the compound represented by the formula (I) above is 1% or more.
[3] The compound according to item [1] or [2] above, which is used for producing a pigment.
[4] The compound according to any one of items [1] to [3] above, in which the isoindoline compound represented by the formula (I) above is an isoindoline compound represented by the following formula (I-1): 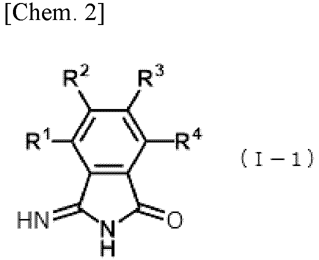 in which, in the formula (I-1), R¹ to R⁴ are as defined for the R¹ to R⁴ in the formula (I).
[5] The compound according to any one of items [1] to [3] above, in which the isoindoline compound represented by the formula (I) above is an isoindoline compound represented by the following formula (I-2): 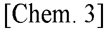 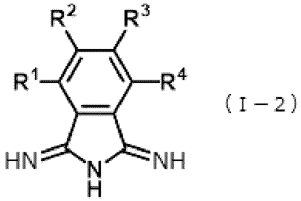 in which, in the formula (I-2), R¹ to R⁴ are as defined for the R¹ to R⁴ in the formula (I).
[6] A composition including the compound according to any one of items [1] to [5] above, which is represented by the formula (I) above.
[7] The composition according to item [6] above, which is used as a composition for a pigment.
[8] The composition according to item [6] above, which contains at least two types of the compounds represented by the formula (I) above.
[9] A method for producing an isoindoline compound, the isoindoline compound containing a radioactive carbon atom ¹⁴C and being represented by the following formula (I): 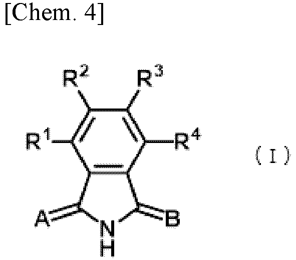 in which, in the formula (I), A and B each independently represent an oxygen atom or NH, and R¹ to R⁴ each independently represent a hydrogen atom, a linear or branched alkyl group having 1 to 12 carbon atoms, a fluorine atom, a chlorine atom, a bromine atom, an iodine atom, or a phenyl group which is optionally substituted with an alkyl group, in which R² and R³ optionally undergo ring closure to form a 5- to 8-membered ring,
   the method including obtaining a compound represented by the following formula (PA) from a compound represented by the following formula (DA): 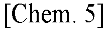 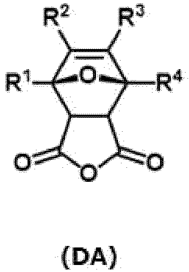 in which, in the formula (DA), R¹ to R⁴ are as defined for the R¹ to R⁴ in the formula (I), 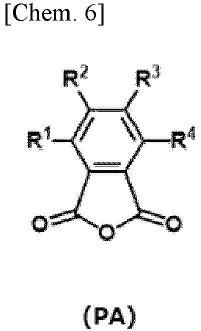 in which, in the formula (PA), R¹ to R⁴ are as defined for the R¹ to R⁴ in the formula (I),
   and obtaining the isoindoline compound represented by the formula (I) above from the compound represented by the formula (PA) above.
[10] The method for producing an isoindoline compound according to item [9] above, in which when obtaining the compound represented by the formula (PA) above from the compound represented by the formula (DA) above, the compound represented by the formula (DA) above is subjected to a reaction under acid conditions formed using an acid selected from the group consisting of hydrochloric acid, sulfuric acid, methanesulfonic acid, p-toluenesulfonic acid, acetic acid, acetic anhydride, triflic acid, phosphoric acid, phosphoric acid anhydride, polyphosphoric acid, pyrophosphoric acid, nitric acid, and a mixture of the acids.
[11] The method for producing an isoindoline compound according to item [9] or [10] above, in which when obtaining the isoindoline compound represented by the formula (I) above from the compound represented by the formula (PA) above, the compound represented by the formula (PA) is subjected to an amination reaction in the presence of ammonium molybdate as a catalyst and urea or NH₃ as an amine source.

### ADVANTAGEOUS EFFECTS OF INVENTION

By the present invention, there can be provided a biomass-derived isoindoline compound (such as KII or DII) which is safe and can be obtained in a clean and carbon neutral approach.

### BRIEF DESCRIPTION OF DRAWINGS

[FIG. 1] FIG. 1 is a drawing showing the results of the GC/MS measurement in Example 1.
[FIG. 2] FIG. 2 is a drawing showing the results of the HPLC measurement in Example 2.

### DESCRIPTION OF EMBODIMENTS

Hereinbelow, the present invention will be described in detail. The following descriptions of the requirements of the invention are examples for explaining the present invention, and should not be construed as limiting the scope of the present invention.

### (Isoindoline compound containing a radioactive carbon atom ¹⁴C)

The isoindoline compound of the invention contains a radioactive carbon atom ¹⁴C.

The isoindoline compound of the invention is represented by the following formula (I).

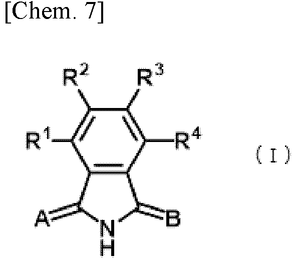

In the formula (I), A and B each independently represent an oxygen atom or NH. R¹ to R⁴ independently represent a hydrogen atom, a linear or branched alkyl group having 1 to 12 carbon atoms, a fluorine atom, a chlorine atom, a bromine atom, an iodine atom, or a phenyl group which is optionally substituted with an alkyl group.

R² and R³ optionally undergo ring closure to form a 5- to 8-membered ring.

The isoindoline compound of the invention is produced using biomass-derived furan and biomass-derived maleic anhydride, and therefore contains a bio (biomass) component derived from a bio raw material. The bio component contained in the isoindoline compound of the invention can be found by, for example, measuring a radioactive carbon atom ¹⁴C.

For example, a biomass-derived compound or composition and petroleum-derived one have no difference therebetween in respect of the molecular weight and physical properties, such as mechanical properties and thermal properties. In order to distinguish these compounds or compositions from one another, generally, a biomass degree is used. The biomass degree is as follows. The carbon of a petroleum-derived compound or composition does not contain ¹⁴C (radioactive carbon 14; half-life: 5,730 years), and therefore the measurement of a ¹⁴C concentration by accelerator mass spectrometry makes it possible to determine whether the formed compound or composition is a petroleum-derived compound or a biomass-derived compound.

The biomass degree is determined as follows. For example, a sample to be measured is burned to generate carbon dioxide, and the carbon dioxide purified using a vacuum-line is reduced with hydrogen using iron as a catalyst to form graphite.

Then, the graphite is set in a ¹⁴C-AMS dedicated apparatus (manufactured by NEC Corporation) composed mainly of a tandem accelerator, and subjected to measurement of ¹⁴C count, ¹³C concentration (¹³C/¹²C), and ¹⁴C concentration (¹⁴C/¹²C), and, from the measured values, a ratio of the ¹⁴C concentration of carbon of the sample to that of standard modern carbon is determined by calculation. In the measurement, oxalic acid (HOxII) provided by the National Institute of Standards and Technology (NIST) can be used as a standard sample.

As an index for evaluating the biomass degree, for example, there can be mentioned pMC (percent modern carbon).

The pMC (percent modern carbon) can be determined by measurement conducted in accordance with ASTM-D6866-18, and indicates a ratio of the ¹⁴C concentration of the sample to be measured to the ¹⁴C concentration of standard modern carbon. When the sample to be measured does not contain a radioactive carbon atom ¹⁴C, the pMC is 0%.

The pMC (percent modern carbon) of the isoindoline compound of the invention represented by the formula (I) is preferably 1% or more, more preferably 50% or more, more preferably 75% or more, more preferably 90% or more, further preferably 99% or more.

In the invention, containing a radioactive carbon atom ¹⁴C means not only that for a segregation approach but also that for a mass balance approach and a book and claim approach (reference document: Enabling Cirrcular Ecoonmy for Chemical with the Mass Balance Approach, the Ellen MacAthur Foundation network).

In the formula (I), as mentioned above, R² and R³ optionally undergo ring closure to form a 5- to 8-membered ring. The formed ring structure can be an ether, alcoholic, or carboxylic functional group. The ring structure which can be an ether, alcoholic, or carboxylic functional group means that the compound may have a -OH group, a -COOH group, or a -O- group in the ring structure. For example, this means that the compound may be a compound represented by the following formula (v), (vi), or (vii).

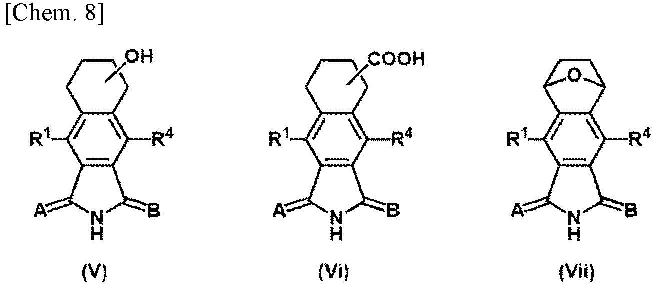

Further, as a more preferred embodiment of the compound represented by the formula (v), (vi), or (vii) above, there can be mentioned a compound represented by the following formula (v'), (vi'), or (vii').

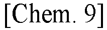

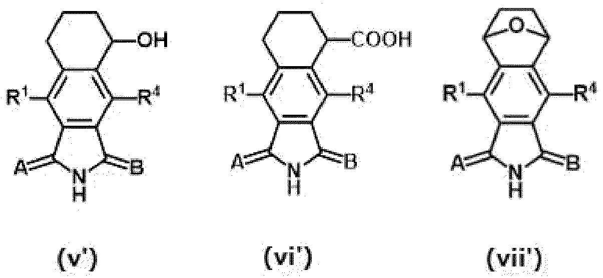

As preferred embodiments of the isoindoline compound represented by the formula (I), for example, there can be mentioned an isoindoline compound represented by the following formula (I-1) and an isoindoline compound represented by the following formula (I-2): 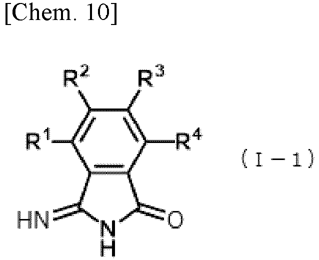 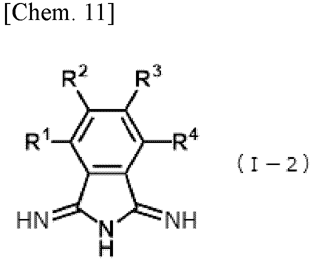 in which, in the formulae (I-1) and (I-2), R¹ to R⁴ are as defined for the R¹ to R⁴ in the formula (I).

Further, as a preferred embodiment of the isoindoline compound represented by the formula (I-1), for example, there can be mentioned KII represented by the following formula (I-1-1), and, as a preferred embodiment of the isoindoline compound represented by the formula (I-2), for example, there can be mentioned DII represented by the following formula (I-2-1).

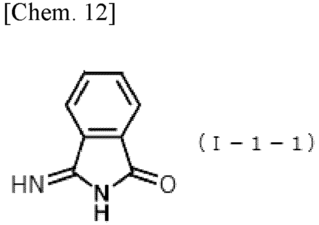

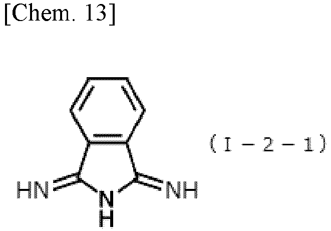

Using the isoindoline compound represented by the formula (I), there can be synthesized various types of isoindoline pigments and dyes and further bioactive isoindolines having properties of an anti-tumor agent, an antioxidant, an anti-fungus agent, or the like.

### (Method for producing an isoindoline compound represented by the formula (I))

The isoindoline compound of the invention represented by the formula (I), which contains a radioactive carbon atom ¹⁴C, i.e., a bio component, can be obtained by the method described below.

From a compound represented by the following formula (DA), a compound represented by the following formula (PA) is obtained: 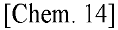 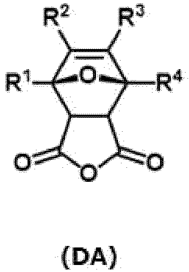 in which, in the formula (DA), R¹ to R⁴ are as defined for the R¹ to R⁴ in the formula (I), 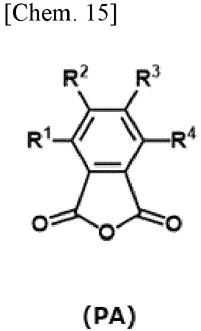 in which, in the formula (PA), R¹ to R⁴ are as defined for the R¹ to R⁴ in the formula (I),
and, from the compound represented by the formula (PA) above, the isoindoline compound represented by the formula (I) above can be obtained.

When obtaining the compound represented by the formula (PA) above from the compound represented by the formula (DA) above, the compound represented by the formula (DA) is subjected to a reaction under acid conditions.

With respect to the acid conditions, any acid conditions can be employed as long as the reaction can advantageously proceed under those conditions, but, for example, the acid conditions are formed using an acid selected from the group consisting of hydrochloric acid, sulfuric acid, methanesulfonic acid, p-toluenesulfonic acid, acetic acid, acetic anhydride, triflic acid, phosphoric acid, phosphoric acid anhydride, polyphosphoric acid, pyrophosphoric acid, nitric acid, and a mixture of the acids.

When obtaining the isoindoline compound represented by the formula (I) above from the compound represented by the formula (PA) above, the compound represented by the formula (PA) is subjected to an amination reaction in the presence of ammonium molybdate as a catalyst and urea or NH₃ as an amine source.

As a preferred embodiment of the method for producing the isoindoline compound represented by the formula (I), there can be mentioned a method for producing the isoindoline compound represented by the formula (I-1) above, and a method for producing the isoindoline compound represented by the formula (I-2) above. The producing methods are individually described below in detail.

### <Method for producing the isoindoline compound represented by the formula (I-1)>

The isoindoline compound represented by the formula (I-1) in the invention can be obtained from a compound represented by the following formula (DA) through a compound represented by the following formula (PA).

A specific reaction path for the method for producing the isoindoline compound is shown below. 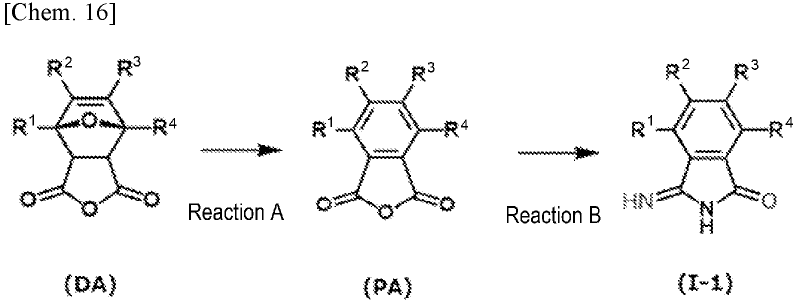 In the formulas, R¹ to R⁴ are as defined for the R¹ to R⁴ in the formula (I).

### <<Reaction A>>

The compound (PA) can be produced by subjecting the compound (DA) to a ringopening dehydration reaction.

With respect to the solvent for a reaction, any solvent can be used as long as it can advantageously advance the reaction, but, for example, there can be mentioned water, acetonitrile, toluene, xylene, an alkylbenzene, a mixed solvent thereof, and non-solvent.

With respect to the reaction temperature, any temperature can be employed as long as it can advantageously advance the reaction, but the reaction temperature is preferably 20 to 150°C, more preferably 30 to 120°C, more preferably 40 to 100°C. The lower limit of the reaction temperature is preferably 20°C or more, preferably 25°C or more, preferably 30°C or more, preferably 35°C or more, preferably 40°C or more. The upper limit of the reaction temperature is preferably 150°C or less, preferably 140°C or less, preferably 130°C or less, preferably 120°C or less, preferably 110°C or less, preferably 100°C or less.

The above-mentioned reaction is conducted under acid conditions. With respect to the acid conditions, any acid conditions can be employed as long as the reaction can advantageously proceed under those conditions, but the acid conditions are formed using an acid selected from the group consisting of hydrochloric acid, sulfuric acid, methanesulfonic acid, p-toluenesulfonic acid, acetic acid, acetic anhydride, triflic acid, phosphoric acid, phosphoric acid anhydride, polyphosphoric acid, pyrophosphoric acid, nitric acid, and a mixture of the acids.

Of these, an acid containing sulfur or phosphorus is preferably used. This is because there is a possibility that such an acid has the δ⁺ and δ⁻ configuration as shown in the following formula (p). 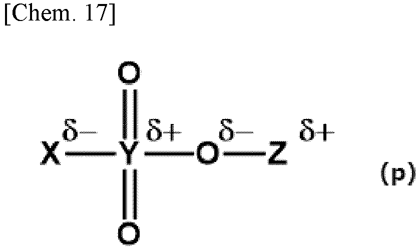 In the formula (p), X represents -OH, -ONa, -OK, or a methyl group, Y represents a sulfur atom or a phosphorus atom, and Z represents a hydrogen atom, -COCH₃, -COH, or - (PO₂)ₙOH.

The amount of the acid, based on the mole of the compound represented by the formula (DA), is preferably 0.1 to 3,000 mol%, preferably 0.5 to 2,500 mol%, preferably 1 to 2,000 mol%, preferably 5 to 1,500 mol%, preferably 10 to 1,000 mol%, preferably 20 to 500 mol%, preferably 50 to 500 mol%, preferably 70 to 500 mol%, preferably 100 to 500 mol%, preferably 150 to 500 mol%, preferably 200 to 500 mol%, preferably 250 to 500 mol%, preferably 300 to 500 mol%. The lower limit of the amount of the acid is preferably 0.1 mol% or more, preferably 0.5 mol% or more, preferably 1 mol% or more, preferably 5 mol% or more, preferably 10 mol%, preferably 20 mol% or more, preferably 50 mol% or more, preferably 70 mol% or more, preferably 100 mol% or more, preferably 150 mol% or more, preferably 200 mol% or more, preferably 250 mol% or more, preferably 300 mol% or more. The upper limit of the amount of the acid is preferably 3,000 mol% or less, preferably 2,500 mol% or less, preferably 2,000 mol% or less, preferably 1,500 mol% or less, preferably 1,000 mol% or less, preferably 500 mol% or less. The upper limit and the lower limit are used in any combination.

### <<Reaction B>>

As shown above in the reaction A, the reaction is conducted under acid conditions and then, in a reaction B, amination is conducted in the presence of ammonium molybdate as a catalyst and urea or NH₃ as an amine source.

In the reaction B, with respect to the replacement of the oxygen atom in the compound represented by the formula (PA) above by a nitrogen atom, when replacing one or two oxygen atoms by a nitrogen atom, there is no particular need to add an additive.

### <Method for producing the isoindoline compound represented by the formula (I-2)>

The isoindoline compound represented by the formula (I-2) in the invention can be obtained from a compound represented by the following formula (DA) through a compound represented by the following formula (PA) or a compound represented by the following formula (I-2 Nitrate).

A specific reaction path for the method for producing the isoindoline compound is shown below. 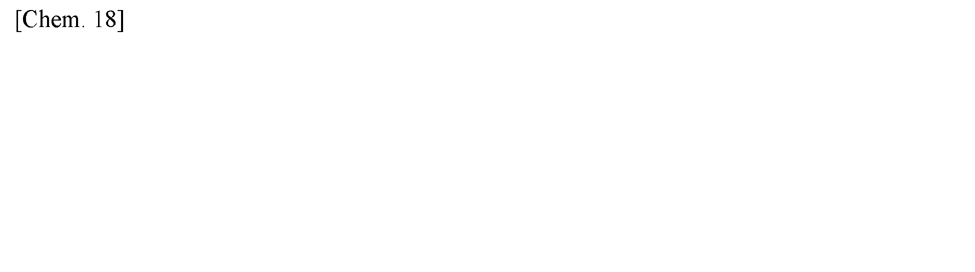 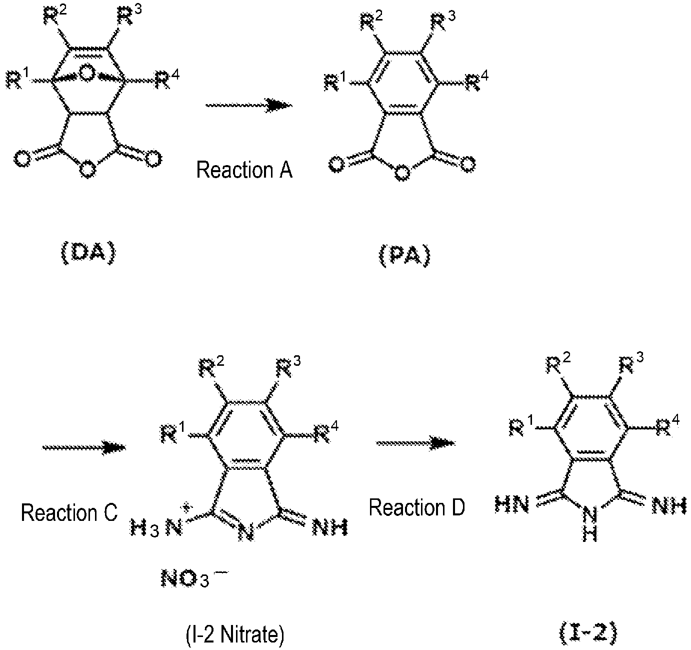 In the formulas, R¹ to R⁴ are as defined for the R¹ to R⁴ in the formula (I).

### <<Reaction A>>

The reaction A is as described above in connection with <<Reaction A>> for the section of <Method for producing an isoindoline compound represented by the formula (I-1)> above.

### <<Reaction C>>

As shown above in the reaction A, the reaction is conducted under acid conditions and then, in a reaction C, amination is conducted in the presence of ammonium molybdate as a catalyst and urea or NH₃ as an amine source.

In the reaction C, with respect to the replacement of the oxygen atom in the compound represented by the formula (PA) above by a nitrogen atom, when replacing one or two oxygen atoms by a nitrogen atom, there is no particular need to add an additive, but, for exchanging all the oxygen atoms by a nitrogen atom as in the reaction C, it is preferred that a compound represented by the following formula (q) is added for preventing a side reaction, such as oligomerization or polymerization.

[Chem. 19] **M^{f+} [NO₃]_{f} (q)**

In the formula (q), M^{f+} represents NH₄⁺, Li⁺, Na⁺, K⁺, Mg²⁺, Ca²⁺, or Al³⁺.

### <<Reaction D>>

In a reaction D above, aqueous ammonia and sodium hydroxide are added to the reaction product obtained through the reaction C to form an isoindoline compound represented by the formula (I-2).

For example, a composition containing the compound represented by the formula (I) is obtained by the above-mentioned method, but, by subjecting the obtained composition to purification according to a method conventionally and commonly used, only the compound represented by the formula (I) (for example, the compound represented by the formula (I-1) or compound represented by the formula (I-2)) can be removed from the composition.

The compound represented by the formula (DA) above used for producing the isoindoline compound of the invention represented by the formula (I) can be obtained by reacting a compound represented by the following formula (FR) and maleic anhydride: 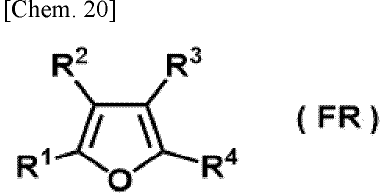 in which, in the formula (FR), R¹ to R⁴ are as defined for the R¹ to R⁴ in the formula (I).

It is preferred that the maleic anhydride is derived from biomass.

It is preferred that the compound represented by the formula (FR) above is derived from biomass.

It is more preferred that both the maleic anhydride and the compound represented by the formula (FR) above are derived from biomass.

The biomass degree of the raw material used in the above-described producing method is preferably 1% or more, more preferably 50% or more, more preferably 75% or more, more preferably 90% or more, further preferably 99% or more. Further, when a biomass raw material prepared in a mass balance system or a book and claim system is available, a biomass degree of 1 to 100% can be more easily achieved. In the present invention, the biomass degree is a biomass degree of the raw material including a biomass raw material prepared in a mass balance system or a book and claim system.

Biomass-derived maleic anhydride can be obtained by subjecting to cyclodehydration or directly oxidizing maleic acid which is obtained by oxidizing FF or HMF obtained by, for example, the method described in JP5791838B or JP6328990B. The compound represented by the formula (FR), which is derived from biomass, can be obtained by subjecting FF or HMF obtained by, for example, the method described in the above-mentioned JP5791838B or JP6328990B to a decarbonylation reaction, a reduction reaction, a dehydration reaction, or the like appropriately in combination.

A specific reaction path for the method for producing the isoindoline compound is shown below.

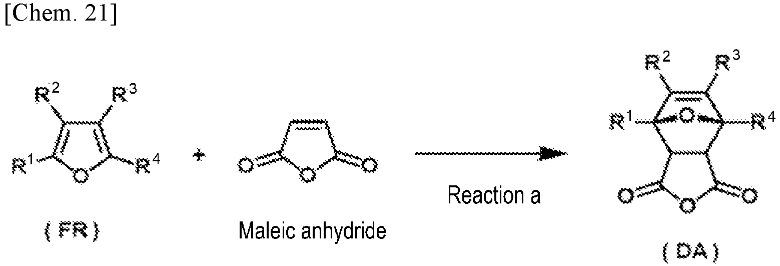

### <<Reaction a>>

The compound (DA) can be produced by subjecting the compound (FR) and maleic anhydride to the Diels-Alder reaction.

The reaction may use a solvent for a reaction, or may be solventless. With respect to the solvent for a reaction, any solvent can be used as long as it can advantageously advance the reaction, but preferred is chloroform, dioxane, ethyl acetate, an alkylbenzene, toluene, xylene, or diethyl ether.

With respect to the reaction temperature, any temperature can be employed as long as it can advantageously advance the reaction, but the reaction temperature is preferably -10 to 100°C, more preferably 0°C to 80°C, further preferably 10°C to 70°C, especially preferably 15°C to 50°C. The lower limit of the reaction temperature is preferably -10°C or more, more preferably 0°C or more, further preferably 10°C or more, especially preferably 15°C or more. The upper limit of the reaction temperature is preferably 100°C or less, more preferably 80°C or less, further preferably 70°C or less, especially preferably 50°C or less.

With respect to the reaction pressure, any pressure can be employed as long as it can advantageously advance the reaction, but the reaction pressure is preferably 0.1 to 5 MPa, more preferably 0.1 to 3 MPa, further preferably 0.1 to 1 MPa, especially preferably 0.1 to 0.5 MPa. The lower limit of the reaction pressure is preferably 0.1 MPa or more, preferably 0.2 MPa or more, preferably 0.3 MPa or more, preferably 0.4 MPa or more. The upper limit of the reaction pressure is preferably 5 MPa or less, preferably 3 MPa or less, preferably 1 MPa or less, preferably 0.9 MPa or less, preferably 0.8 MPa or less, preferably 0.7 MPa or less, preferably 0.6 MPa or less, preferably 0.5 MPa or less.

### (Composition including the isoindoline compound)

The invention may be a composition containing one type or two types or more of the isoindoline compounds of the invention represented by the formula (I).

As a preferred embodiment of the composition of the invention, for example, there can be mentioned a composition including an isoindoline compound represented by the following formula (I-1), a composition including an isoindoline compound represented by the following formula (I-2), a composition including the isoindoline compound represented by the following formula (I-1) and the isoindoline compound represented by the following formula (I-2), and a composition including the isoindoline compound represented by the following formula (I-1) and an isoindoline compound represented by the following formula (I-3).

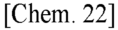

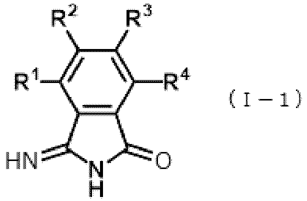

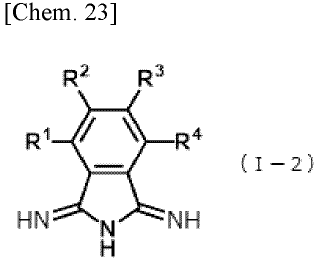

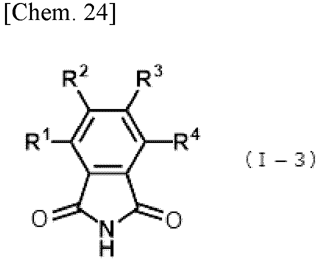

In the formulae (I-1), (I-2), and (I-3), R¹ to R⁴ are as defined for the R¹ to R⁴ in the formula (I).

The isoindoline compound represented by the formula (I-1) above or the isoindoline compound represented by the formula (I-2) above may undergo an isomerization reaction as shown in the following formula (s) to form an isoindoline compound represented by the following formula (i) or (ii), or may undergo an isomerization reaction as shown in the following formula (t) to form an isoindoline compound represented by the following formula (iii) or (iv).

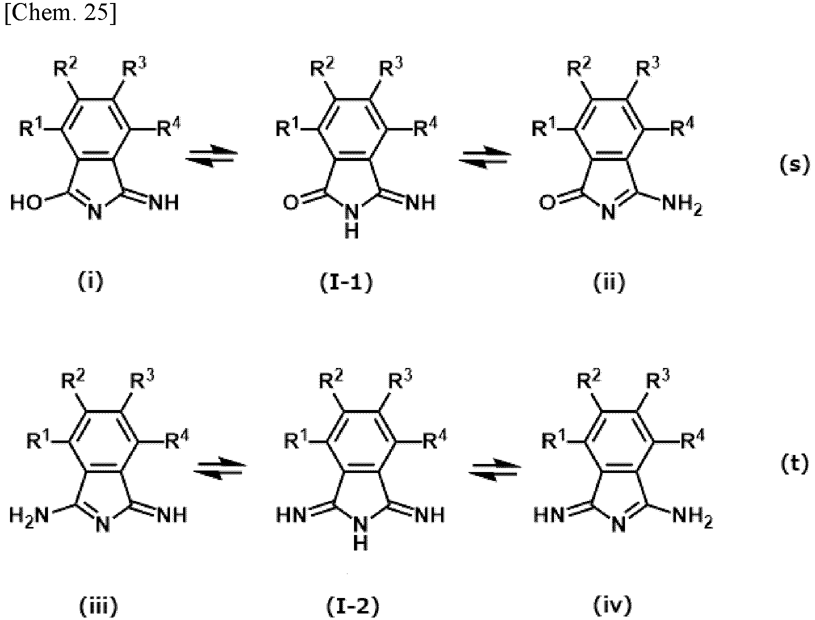

In the formulae (i) to (iv), R¹ to R⁴ are as defined for the R¹ to R⁴ in the formula (I).

Therefore, the composition of the invention may be, for example, a composition including the isoindoline compound represented by the formula (I-1) above and the isoindoline compound represented by the formula (i) or (ii) above, or a composition including the isoindoline compound represented by the formula (I-2) above and the isoindoline compound represented by the formula (iii) or (iv) above.

Using the composition, a composition for a pigment can be prepared.

### (Application of the isoindoline compound of the invention)

The isoindoline compound of the invention obtained by the above-described method, which contains a radioactive carbon atom ¹⁴C and which is represented by the formula (I), is an important intermediate for producing a pigment. By using the isoindoline compound of the invention, various excellent pigments, such as Y173, Y139, and Y185, can be produced.

Further, using the isoindoline compound of the invention, various excellent bioactive isoindolines having properties of an anti-tumor agent, an antioxidant, an anti-fungus agent, or the like can be produced.

The isoindoline compound of the invention contains biomass-derived carbon and thus is carbon neutral and contributes to reduction of a burden on the environment.

Further, the isoindoline compound of the invention is obtained by the above-described method, and therefore, by changing the type of furan or changing the furan to another type of diene, the number and position of various types of functional groups can be controlled.

As mentioned above, the isoindoline compound of the invention can be applied to various uses.

For example, a pigment is produced using the isoindoline compound of the invention and a composition having the isoindoline compound, and the produced pigment which, if necessary, has mixed an additional resin, rubber, additive, pigment, dye, or the like is prepared and used for a cosmetic, pharmaceutical or agricultural coating material or printing marker, stationery, a writing utensil, a printing ink, an inkjet ink, a metallic ink, a coating composition, a plastic colorant, a color toner, a color filter, and the like.

### EXAMPLES

Hereinbelow, the invention will be described in more detail with reference to the following Examples, which should not be construed as limiting the scope of the invention. In the following Examples, the unit "%" used for the composition means "% by mass".

### (Synthesis Example 1) Synthesis of a DA intermediate

In a reactor equipped with a stirrer in a nitrogen gas atmosphere, 12 g of biomass-derived maleic anhydride was dissolved in diethyl ether (250 mL), and 10 g of biomass-derived furan was charged and the resultant mixture was subjected to a reaction at room temperature under 0.25 MPa for 24 hours. Then, the resultant white crude product was taken by filtration using a filter, and subjected to vacuum drying to obtain 20 g of a DA intermediate.

### (Example 1) Bio KII

14 g of acetic anhydride was added to 74 g of methanesulfonic acid, and 5 g of the DA intermediate was slowly added to the resultant mixture while cooling in an ice bath. The reaction mixture was stirred at room temperature for 2 hours, and then heated at 80°C for 4 hours. The resultant reaction mixture was subjected to extraction with toluene (3 x 50 mL). The collected toluene was washed with an aqueous sodium chloride solution and sodium hydrogencarbonate, and evaporated under a reduced pressure. Into the resultant white needles in 10 mL of an alkylbenzene were mixed 5.3 g of urea and 0.02 g of molybdenum. The reaction mixture was heated at 150°C for 2 hours. The resultant mixture was cooled to room temperature, and then subjected to filtration, and washed with ethyl acetate and ethanol. The resultant solids were subjected to vacuum drying, obtaining 2.1 g of a compound containing radioactive carbon C¹⁴ in the molecule thereof and having a purity of 77%.

### (Example 2) Bio DII

14 g of acetic anhydride was added to 74 g of methanesulfonic acid, and 5 g of the DA intermediate was slowly added to the resultant mixture while cooling in an ice bath. The reaction mixture was stirred at room temperature for 2 hours, and then heated at 80°C for 4 hours. The resultant reaction mixture was subjected to extraction with toluene (3 x 50 mL). The collected toluene was washed with an aqueous sodium chloride solution and sodium hydrogencarbonate, and evaporated under a reduced pressure. Into the resultant white needles in 10 mL of an alkylbenzene were mixed 13 g of urea, 6 g of ammonium nitrate, and 0.03 g of molybdenum. The reaction mixture was heated at 165°C for 5 hours, and then the alkylbenzene was removed by vacuum distillation. 30 mL of methanol was added and the resultant mixture was subjected to distillation for 15 minutes, and then cooled to room temperature and subjected to filtration. The resultant solids were dispersed in 10 mL of water, and 3 g of aqueous ammonia and 4.4 mL of 25% caustic soda were added. The solution was cooled in an ice bath, and the deposited solids were subjected to filtration and washed with water. The resultant solids were subjected to vacuum drying, obtaining 2.9 g of a compound containing radioactive carbon C¹⁴ in the molecule thereof and having a purity of 100%.

### (Comparative Example 1) Synthesis of petroleum-derived KII

In accordance with the method described in Japanese Patent Application No. H05-264068, petroleum-derived KII was synthesized.

### (Comparative Example 2) Petroleum-derived DII

A commercially available DII (manufactured by Tokyo Chemical Industry Co., Ltd.) was purchased and used.

### (Gas chromatography/mass spectrometry)

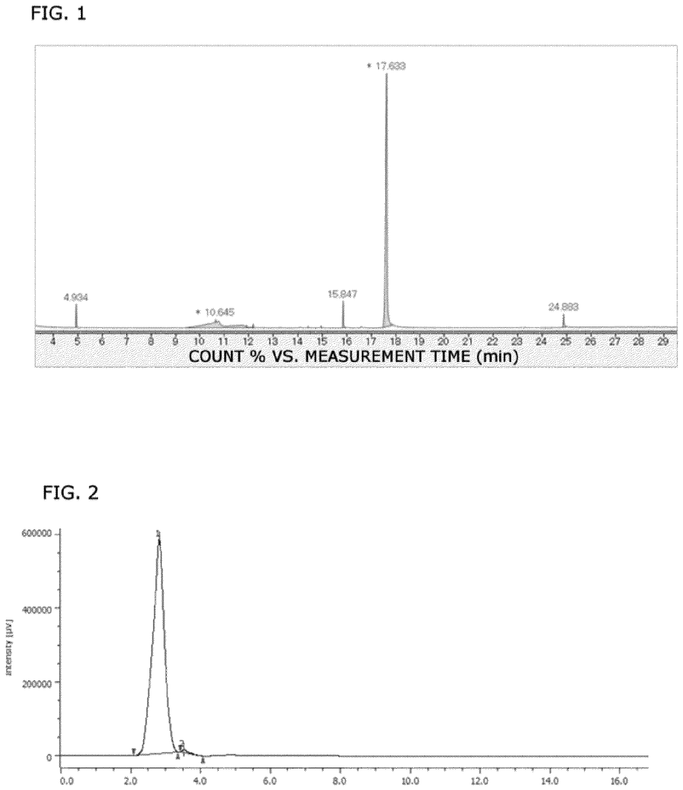

With respect to the isoindoline compound (KII) obtained in Example 1 above, gas chromatography/mass spectrometry analysis was conducted. A method for the analysis was such that about 2.5 mg of a measurement sample was placed in a measuring flask, and THF (containing no antioxidant) was added so that the amount of the resultant solution became 5 mL, obtaining a solution for measurement, and GC/MS (8890 GC System and 5977BGC-MSD, manufactured by Agilent Technologies, Inc.) measurement of the solution was conducted. The results of the measurement are shown in FIG. 1.

### (High performance liquid chromatography)

With respect to the isoindoline compound (DII) obtained in Example 2 above, high performance liquid chromatography analysis was conducted. A method for the analysis was such that 5 mg of a measurement sample was dissolved in 10 mL of acetonitrile, and the resultant solution was subjected to filtration, and HPLC measurement of the solution was conducted. The results of the measurement are shown in FIG. 2. In FIG. 2, tR was as follows: tR = 2.820. tR of commercially available DII is as follows: tR = 2.853.

### (Accelerator mass spectrometry (AMS method))

Using a ¹⁴C-AMS dedicated apparatus (manufactured by NEC Corporation) composed mainly of a tandem accelerator, measurement of ¹⁴C count, ¹³C concentration (¹³C/¹²C), and ¹⁴C concentration (¹⁴C/¹²C) were conducted.

In the measurement, oxalic acid (HOxII) provided by the National Institute of Standards and Technology (NIST) was used as a standard sample. The measurement for the standard sample and a background sample was simultaneously conducted. The pMC (percent Modern Carbon) is a ratio of the ¹⁴C concentration of carbon of the sample to that of standard modern carbon.

The results of the analysis by an AMS method in Examples 1 and 2 and Comparative Examples 1 and 2 are shown in Table 1 below.

**[Table 1]**

| Sample name | pMC (%) |
|---|---|
| Example 1 | 77.82 ± 0.23 |
| Example 2 | 100.44 ± 0.29 |
| Comparative Example 1 | 0.64 ± 0.03 |
| Comparative Example 2 | <0.13 |

## Claims

1. An isoindoline compound containing a radioactive carbon atom ¹⁴C and being represented by the following formula (I): 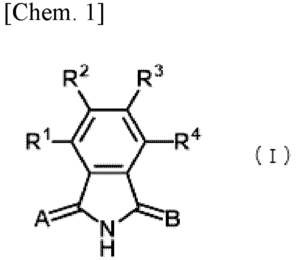 wherein, in the formula (I), A and B each independently represent an oxygen atom or NH, and R¹ to R⁴ each independently represent a hydrogen atom, a linear or branched alkyl group having 1 to 12 carbon atoms, a fluorine atom, a chlorine atom, a bromine atom, an iodine atom, or a phenyl group which is optionally substituted with an alkyl group, wherein R² and R³ optionally undergo ring closure to form a 5- to 8-membered ring.

2. The compound according to claim 1, wherein the pMC (percent modern carbon) of the compound represented by the formula (I) above is 1% or more.

3. The compound according to claim 1, which is used for producing a pigment.

4. The compound according to claim 1, wherein the isoindoline compound represented by the formula (I) above is an isoindoline compound represented by the following formula (I-1): 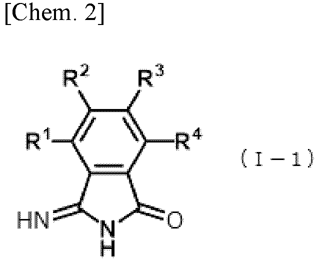 wherein, in the formula (I-1), R¹ to R⁴ are as defined for the R¹ to R⁴ in the formula (I).

5. The compound according to claim 1, wherein the isoindoline compound represented by the formula (I) above is an isoindoline compound represented by the following formula (I-2): 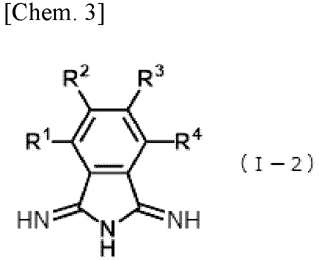 wherein, in the formula (I-2), R¹ to R⁴ are as defined for the R¹ to R⁴ in the formula (I).

6. A composition comprising the compound according to claim 1, which is represented by the formula (I) above.

7. The composition according to claim 6, which is used as a composition for a pigment.

8. The composition according to claim 6, which contains at least two types of the compounds represented by the formula (I) above.

9. A method for producing an isoindoline compound, the isoindoline compound containing a radioactive carbon atom ¹⁴C and being represented by the following formula (I): 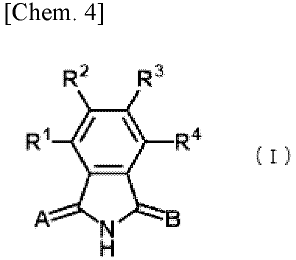 wherein, in the formula (I), A and B each independently represent an oxygen atom or NH, and R¹ to R⁴ each independently represent a hydrogen atom, a linear or branched alkyl group having 1 to 12 carbon atoms, a fluorine atom, a chlorine atom, a bromine atom, an iodine atom, or a phenyl group which is optionally substituted with an alkyl group, wherein R² and R³ optionally undergo ring closure to form a 5- to 8-membered ring,
the method comprising obtaining a compound represented by the following formula (PA) from a compound represented by the following formula (DA): 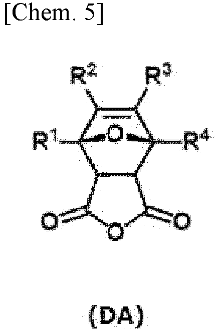 wherein, in the formula (DA), R¹ to R⁴ are as defined for the R¹ to R⁴ in the formula (I), 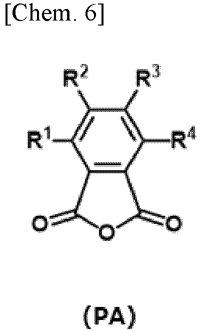 wherein, in the formula (PA), R¹ to R⁴ are as defined for the R¹ to R⁴ in the formula (I),
and obtaining the isoindoline compound represented by the formula (I) above from the compound represented by the formula (PA) above.

10. The method for producing an isoindoline compound according to claim 9, wherein when obtaining the compound represented by the formula (PA) from the compound represented by the formula (DA), the compound represented by the formula (DA) is subjected to a reaction under acid conditions formed using an acid selected from the group consisting of hydrochloric acid, sulfuric acid, methanesulfonic acid, p-toluenesulfonic acid, acetic acid, acetic anhydride, triflic acid, phosphoric acid, phosphoric acid anhydride, polyphosphoric acid, pyrophosphoric acid, nitric acid, and a mixture of the acids.

11. The method for producing an isoindoline compound according to claim 9, wherein when obtaining the isoindoline compound represented by the formula (I) from the compound represented by the formula (PA), the compound represented by the formula (PA) is subjected to an amination reaction in the presence of ammonium molybdate as a catalyst and urea or NH₃ as an amine source.
